(19) Europäisches Patentamt
European Patent Office
Office européen des brevets

(11) EP 0 849 268 B1

(12) # EUROPÄISCHE PATENTSCHRIFT

(45) Veröffentlichungstag und Bekanntmachung des Hinweises auf die Patenterteilung:
**10.10.2001 Patentblatt 2001/41**

(51) Int Cl.[7]: **C07D 498/04**, C08G 18/79
// (C07D498/04, 273:00, 243:00)

(21) Anmeldenummer: **97121543.9**

(22) Anmeldetag: **08.12.1997**

(54) **Polycyclische Iminooxadiazindione aus (cyclo)aliphatischen 1,4-Diisocyanaten**

Polycyclic iminooxadiazindiones from (cyclo)aliphatic 1,4-diisocyanates

Iminooxadiazindiones polycycliques à partir de 1,4-diisocyanates (cyclo)aliphatiques

(84) Benannte Vertragsstaaten:
**DE ES FR GB IT NL SE**

(30) Priorität: **20.12.1996 DE 19653583**

(43) Veröffentlichungstag der Anmeldung:
**24.06.1998 Patentblatt 1998/26**

(73) Patentinhaber: **BAYER AG**
**51368 Leverkusen (DE)**

(72) Erfinder:
- **Richter, Frank, Dr.**
  **51373 Leverkusen (DE)**
- **Mager, Dieter**
  **51377 Leverkusen (DE)**
- **Pedain, Josef, Dr.**
  **51061 Köln (DE)**

(56) Entgegenhaltungen:
**EP-A- 0 571 038** **DE-A- 3 739 549**
**DE-A- 3 902 078** **DE-A- 19 532 060**
**DE-A- 19 611 849**

- **B. AKTERIES ET AL.: CHEMISCHE BERICHTE, Bd. 119, 1986, Seiten 1133-43, XP002059955**
- **K. H. SLOTTA ET AL.: BERICHTE DER DEUTSCHEN CHEMISCHEN GESELLSCHAFT, Bd. 60, Nr. 2, 1927, Seiten 295-301, XP002059956**
- **K. H. SLOTTA ET AL.: BERICHTE DER DEUTSCHEN CHEMISCHEN GESELLSCHAFT, Bd. 60, Nr. 4, 1927, Seiten 1011-1020, XP002059957**
- **A. ETIENNE ET AL.: COMPT. REND. ACAD. SC. PARIS, Bd. C 277, 1973, Seiten 795-8, XP002034781**
- **E. SCHAUMANN ET AL.: CHEMISCHE BERICHTE, Bd. 120, 1987, Seiten 339-44, XP002059958**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Die Erfindung betrifft polycyclische Iminooxadiazindione aus 1,4-Diisocyanaten.

Die Reaktion von Isocyanaten mit einer außerordentlichen Vielzahl H-acider Verbindungen als Aufbauprinzip hochmolekularer Stoffe basierend auf den Arbeiten von O. Bayer et al. ist bekannt (z.B. DE-A 728 981) und hat breite industrielle Anwendung gefunden. Hierbei macht man sich die hohe Reaktivität der Isocyanatgruppierung gegenüber den o.g. Reaktanden zunutze.

Die unangenehmen physiologischen Eigenschaften niedermolekularer (Di)isocyanate verhindern mitunter ihren unmittelbaren Einsatz, z.B. auf dem Lacksektor. Deshalb bedient man sich verschiedener Verfahren zur Modifizierung dieser Monomeren mit dem Ziel, den Dampfdruck der resultierenden Produkte wesentlich zu erhöhen und mithin zu physiologisch unbedenklichen Verbindungen zu gelangen. Beispiele hierfür sind die anteilige Umsetzung von Diisocyanaten mit zwei- und höherwertigen Alkoholen (Prepolymerisierung) sowie die Herstellung von Polyisocyanaten mit Uretdion-("Dimer-"), Isocyanurat-("Trimer-") sowie Biuretstrukturelementen.

Weiterhin ist es mitunter erforderlich, NCO-Gruppen mit verringerter Reaktivität einzusetzen (1K-Anwendungen mit möglichst langer Topfzeit, Polyurethan-Pulverlacksysteme, wäßrige Systeme usw.). Um zu Produkten zu gelangen, die in diesen Anwendungen eingesetzt werden können, muß man eine thermisch oder chemisch reversible Blockierung mit Substraten vornehmen, die bei der Vernetzungsreaktion zum polymeren Kunststoff oder Lackfilm wieder, gegebenenfalls in veränderter Form, abgespalten werden (z.B. Progr. Org. Coatings, 3 (1975), 73 und 9 (1981), 3). Hierfür haben sich eine Reihe von Verbindungen in der Praxis bewährt, wie z.B. ε-Caprolactam, Malonsäuredialkylester sowie Butanonoxim.

Der Nachteil all dieser Systeme besteht darin, daß die Blockierungsmittel bei der Aushärtungsreaktion, gegebenenfalls in veränderter Form, abgespalten werden und entweder entsorgt werden müssen oder, sofern sie im Kunststoff oder der Beschichtung verbleiben, das Eigenschaftsprofil letzterer nachteilig beeinflussen, z.B. infolge "Ausschwitzens" oder einer Verschlechterung der physikalischen und/oder chemischen Beständigkeit der Produkte.

In gewisser Hinsicht eine Ausnahme stellen in diesem Zusammenhang die bereits erwähnten Lackpolyisocyanate auf Uretdionbasis ("Dimerisate") dar, die sich, im Gegensatz zu vielen anderen Isocyanat-Folgeprodukten thermisch induziert in die (Ausgangs)isocyanate rückspalten lassen. Der Nachteil des Zwangsanfalls eines bei der Aushärtung freiwerdenden Abbauproduktes besteht bezüglich dieser Spaltung nicht.

Aus dieser thermischen Labilität der Uretdionstruktur können jedoch mitunter einige Nachteile resultieren. Einerseits kann die längere Lagerung bei höherer Temperatur zu einer langsamen Aufspaltung der Uretdionringe führen, die in einer Erhöhung des Restmonomerengehaltes und demzufolge physiologisch nicht mehr einwandfreien Produkten resultiert. Andererseits erfolgt die zur schnellen Aushärtung des Kunststoffes bzw. der Beschichtung notwendige thermisch induzierte Dissoziation der Uretdionringe erst bei relativ hohen Temperaturen, was zu Verfärbungen und anderen unerwünschten Zersetzungserscheinungen führen kann.

Weiterhin ist in diesem Zusammenhang die Verwendung der, z.B. nach der Lehre der EP-A 14 365 herstellbaren, ähnlich den Uretdionen sozusagen "selbstblockierten" Verbindungen vom sog. α-Nylon Typ erwähnenswert. Allerdings setzt die zur Vernetzung nötige Spaltung dieser Polymerisate, sofern sie überhaupt gelingt, erst bei noch höheren Temperaturen ein, als das bei den Uretdionen der Fall ist, weshalb ihr Einsatz insbesondere auf dem Lacksektor bisher nicht erfolgte.

Aufgabe der vorliegenden Erfindung war es daher, Systeme zu entwickeln, bei denen kein, oder zumindest ein im Vergleich zu den herkömmlichen, Blockierungsmittel enthaltenden Systemen des Standes der Technik deutlich verringerter Anteil an Isocyanatgruppen durch Zugabe eines Blockierungsmittels desaktiviert werden muß und trotzdem, auch bei höherer Temperatur, keine Rückspaltung in (monomeres) (Di)isocyanat einsetzen sollte.

Diese Aufgabe konnte durch die Bereitstellung der erfindungsgemäßen bi- und polycyclischen Iminooxadiazindione (im folgenden: polycyclische Iminooxadiazindione) gelöst werden.

Gegenstand der vorliegenden Erfindung sind polycyclische Iminooxadiazindione der allgemeinen Formel (I) A

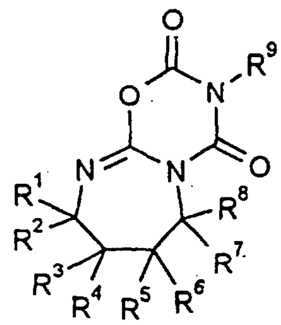

(I),

A

in denen $R^1$-$R^8$ für gleiche oder verschiedene Reste wie: H, Alkyl, Cycloalkyl, Aralkyl und Aryl, die ihrerseits funktionelle Substituenten tragen können, die nicht mit Isocyanatgruppen reagieren, wie z.B. Isocyanato(cyclo)alkyl, Halogen, Oxo (-C(=O)-), Alk- oder Aryl-oxi (R-O-), Alkoxycarbonyl (ROC(=O)-), Aroyl (RC(=O)-) usw., wobei auch $R^1$ und/oder $R^2$ untereinander sowie mit einem oder mehreren Substituenten aus der Reihe $R^3$ - $R^8$ sowie den entsprechenden Atomen des Siebenringsegmentes in A einen oder mehrere, gegebenenfalls partiell ungesättigte, Ringe bilden können und $R^3$ oder $R^4$ mit wahlweise $R^5$ oder $R^6$ sowie den beiden nicht stickstoffgebundenen Kohlenstoffatomen des Siebenringsegmentes in A einen aromatischen Ring bilden können und schließlich $R^9$ beschrieben wird durch: H, Alkyl, Cycloalkyl, Aralkyl, Aryl, funktionell substituierte (cyclo)aliphatische, heterocyclische und/oder aromatische Reste die z.B. eine oder mehrere Isocyanatgruppen und/oder andere funktionelle Substituenten, die nicht mit Isocyanaten reagieren, wie z.B. Halogen, Oxo (-C(=O)-), Alk- oder Aryl-oxi (R-O-), Alkoxycarbonyl (ROC(=O)-), Aroyl (RC(=O)-) usw. enthalten können.

Gegenstand der Erfindung sind weiterhin polycyclische Iminooxadiazindione A der Formel (I) in denen $R^1$-$R^8$ die oben beschriebene Bedeutung haben und $R^9$ für einen, gegebenenfalls substituierten, gegebenenfalls Heteroatome enthaltenden Alkyl-, Cycloalkyl-, Aralkyl- oder Arylsubstituenten B der Formel (II) steht.

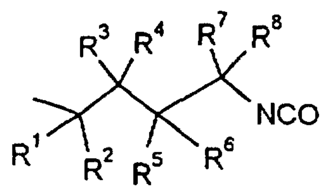

(II)

B

Gegenstand der Erfindung sind weiterhin Gemische der genannten polycyclischen Iminooxadiazindione mit anderen Isocyanat-Folgeprodukten des Standes der Technik wie z.B. Isocyanuraten, Uretdionen, Biureten, Allophanaten, Carbodiimiden sowie Urethanen, die ihrerseits weitere Isocyanatgruppen, letztere gegebenenfalls in blockierter Form, enthalten können.

Gegenstand der Erfindung ist ferner ein Verfahren zur Herstellung der vorstehend genannten Verbindungen bzw. Gemische durch katalytisch induzierte Reaktion eines Di-oder Oligoisocyanates C der Formel (III)

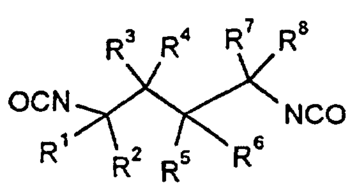

(III)

C

in dem mindestens zwei Isocyanatgruppen als Substituenten an einem, gegebenenfalls substituierten, gegebenenfalls Heteroatome enthaltenden, gegebenenfalls cyclischen, (ar)aliphatischen System in 1,4-Stellung zueinander angeordnet sind, mit mindestens einem gleichen oder anderen, Mono- oder Polyisocyanat.

Die Erfindung betrifft ferner die Verwendung der erfindungsgemäßen polycyclischen Iminooxadiazindione als Zwischenprodukt sowie als Komponente zur Herstellung von, gegebenenfalls geschäumten, Polyurethankunststoffen, Lacken und Beschichtungsmitteln und zur Herstellung oder Formulierung von Wirkstoffen, pharmazeutischen Erzeugnissen etc..

Der Erfindung liegt die überraschende Beobachtung zugrunde, daß 1,4-Diisocyanate, gegebenenfalls in Gegenwart anderer Mono- und/oder Polyisocyanate, katalytisch induziert in die erfindungsgemäßen polycyclischen Iminooxadiazindione überführt werden können.

Dieser Befund ist deshalb sehr überraschend, da aus der Literatur bekannt ist, daß 1,4-Diisocyanate in einem weiten Temperaturbereich bei Einsatz verschiedener Katalysatoren einerseits zur Oligomerisierung ('Trimerisierung' unter Ausbildung von Isocyanuratstrukturen vgl. z.B. EP 571 038, 'Dimerisierung' unter Ausbildung von Uretdionstrukturen) und andererseits, häufig gleichzeitig, zur (Cyclo)polymerisation unter Bildung hochmolekularer Verbindungen vom $\alpha$-Nylon Typ tendieren (ein Übersichtsartikel hierzu mit weiteren Literaturzitaten findet sich z.B. in: R. J. Cotter and M. Matzner, "Organic Chemistry, A Series of Monographs", part 2, vol. 13B2, S. 332 ff., vgl. auch Beispiel 1). Wie bereits eingangs erwähnt, sind insbesondere letztere Spezies mit gravierenden Mängeln, beispielsweise hinsichtlich einer Verwendung in Beschichtungsmitteln, behaftet.

Die Bildung von polycyclischen Iminooxadiazindionen wurde in keinem Falle beobachtet.

Es finden sich ganz allgemein in der Literatur nur wenige Zitate, in denen die Bildung von Iminooxadiazindionverbindungen durch katalytisch induzierte Modifizierung von Isocyanaten beschrieben wird.

Slotta und Tschesche erhielten das monocyclische Trimethylderivat (3,5-Dimethyl-2-methylimino-4,6,diketo-1,3,5-oxadiazin), neben anderen Produkten, bei der Umsetzung von Methylisocyanat in Gegenwart von Tributylphosphin (Chem. Ber., 60 (1927), 295 und 1011). Diese Verbindung soll in besserer Ausbeute zugänglich sein, wenn in 1,2-Dichlorethan als Lösungsmittel gearbeitet wird (C. R. Acad. Sci. Ser. C 277 (1973) 795). Das formal aus einem Equivalent Phenyl- und zwei Equivalenten Methylisocyanat aufgebaute 3-Phenyl-5-methyl-2-methylimino-4,6-diketo-1,3,5-oxadiazin entsteht, neben anderen Produkten, bei der Reaktion von Diphenyl-methylimidocarbonat mit Tosylisocyanat (Chem. Ber. 120 (1987), 339). In der DE-A 3 902 078 wird die Bildung des Tris(6-isocyanatohexyl)derivates (3,5-Bis(6-isocyanatohexyl)-2-(6-isocyanatohexyl)imino-4,6-diketo-1,3,5-oxadiazin) in untergeordnetem Maße bei der Trimerisierung von Hexamethylendiisocyanat in Gegenwart von Kohlendioxid am Rande erwähnt. Diese Verbindungen lassen sich nach der Lehre der DE-A 19 611 849 gezielt herstellen.

Polycyclische Verbindungen mit Iminooxadiazindionstruktur waren unlängst noch gänzlich unbekannt. Vertreter, die ausgehend von 1,3-Diisocyanaten zugänglich sind, werden in der DE-A 19 532 060 beschrieben.

Die erfindungsgemäßen polycyclischen Iminooxadiazindione lassen sich durch katalytisch induzierte Reaktion eines Poly-, bevorzugt Diisocyanat(gemisch)es, wobei gegebenenfalls zusätzlich auch andere Mono- oder Polyisocyanate als Reaktionspartner verwendet werden können, herstellen, bei dem in mindestens einer der Ausgangskomponenten zwei Isocyanatgruppen als Substituenten an einem, gegebenenfalls substituierten, gegebenenfalls Heteroatome enthaltenden, gegebenenfalls cyclischen, (ar)aliphatischen System in 1,4-Stellung zueinander angeordnet sind.

Zur Herstellung der erfindungsgemäßen polycyclischen Iminooxadiazindione setzt man als 1,4-Diisocyanate, gegebenenfalls im Gemisch mit anderen Mono- und Polyisocyanaten sowie, wo dies möglich ist, auch als Stereoisomerengemische untereinander, Verbindungen des Molekulargewichtsbereiches von 140 bis 800, besonders bevorzugt 140-400 Dalton ein. Beispiele für derartige, literaturbekannte 1,4-Diisocyanate sind: 1,4-Diisocyanatobutan ('Butandiisocyanat', BDI), 2,2,3,3-Tetrafluor-1,4-butandiisocyanat, 2-Chlor-1,4-diisocyanatobutan, 2,3-Dibrom-1,4-diisocyanatobutan, 2,3-Bis(difluoramino)-1,4-diisocyanatobutan, 1,4-Diisocyanato-1,1,2,2,3,3,4,4-octafluorbutan, 1,4-Diisocyanato-1-propoxybutan, 1,4-Diisocyanatopentan, 1,4-Diisocyanato-4-methylpentan, 2,5-Diisocyanatohexan, 3,6-Diisocyanatooctan, 1-Isocyanato-3-(isocyanatomethyl)pentan, 2,5-Diisocyanato-2,5-dimethylhexan, 1-Isocyanato-3-(isocyanatomethyl)-3,5,5-trimethylhexan, 1,3,6-Triisocyanatohexan, 2,5,8-Triisocyanatooctan, 1,4-Diisocyanato-1-buten, 1,4-Diisocyanato-2-buten, 2-Butandioyldiisocyanat, 2-Butendioyldiisocyanat, 4,7-Diisocyanato-2,6,6-trimethyl-2-hepten, 1-Methylen-1,4-butandiisocyanat, 2,5-Diisocyanatopentansäuremethyl-, ethyl-, propyl-, benzyl-, butyl-, hexyl-, octyl-, decyl-, dodecyl- sowie octadecyl-ester, 2,5-Diisocyanatohexansäuremethylester, 2,5-Diisocyanatoheptansäuremethylester, 2,5-Diisocyanatohexandisäuredi-methyl-, ethyl-, sowie butyl-ester, 1,2-Bis(isocyanatomethyl)-cyclobutan, 1,4-Diisocyanatocyclohexan, 1,4-Diisocyanato-2-methylcyclohexan 1,2-Bis(isocyanatomethyl)-cyclohexan, 5,6-Bis(isocyanatomethyl)-bicyclo[2.2.1]hept-2-en, 1,4-Diisocyanatobicyclo[2.2.2.]octan, 2-Isocyanato-6-isocyanatomethylpyran, 1,1-Dimethyl-3-isocyanato-5-isocyanatomethylcyclohexan, 2-Isocyanato-4-isocyanatomethyl-1,1,4-trimethylcyclohexan, 2-Isocyanato-4-isocyanatomethyl-1,1-dimethylcyclobutan, 1,4-Diisocyanato-2,3,5,6-tetramethylcyclohexan, 1-Isocyanato-3-isocyanatomethyl-1-methylcyclohexan (gegebenenfalls auch in Abmischung mit den 2 isomeren 1-Isocyanato-4-isocyanatomethyl-1-methylcyclohexan, 'techn IMCI'), 2-Isocyanato-4-isocyanatomethyl-1-methylcyclohexan, 5-Isocyanato-1-isocyanatomethyl-1,3,3-trimethylcyclohexan ('IPDI'), 2,5-Diisocyanato-1,1,3-trimethylcyclohexan, 1-Isocyanato-3-isocyanatomethylcyclohexan, 1-Isocyanato-3-(1-isocyanatoethyl)cyclohexan, 1-Isocyanato-3-(1-isocyanatopropyl)cyclohexan, 4-Cyclohexen-1,2-dicarbonyldiisocyanat, 1,2-Bis(isocyanatomethyl)-benzol ('Xylylendiisocyanat', XDI), 1,2-Bis(1-isocyanato-1-methylethyl)-benzol ('Tetramethylxylylendiisocyanat', TMXDI), 4,5-Bis(isocyanatomethyl)-1,3-dithiolan, 4,5-Bis(isocyanatomethyl)-2-methyl-1,3-dithiolan, 2-(2-Isocyanatoethyl)-2-(isocyanatomethyl)-1,3-dithiolan, 2-(2-Isocyanatoethyl)-2-(isocyanatomethyl)-1,3-dioxolan 3,4-Bis(isocyanatomethyl)-tetrahydrothiophen, 2,3-Bis(isocyanatomethyl)-1,4-dithian, 1,2,3,4-Tetramethoxy-1,4-butandiisocyanat und 1,4-Diisocyanato-1,2,3,4-butantetroltetraacetat

Bei diesen 1,4-Diisocyanaten handelt es sich um literaturbekannte Verbindungen, die nach etablierten Verfahren des Standes Technik, mit oder ohne Verwendung von Phosgen, z.B. aus den zugrundeliegenden Diaminen oder auf anderen Wegen (Curtius-Abbau, Urethanspaltung etc.) hergestellt werden können.

Da, insbesondere bei Einsatz cycloaliphatischer 1,4-Diisocyanate und hier insbesondere bei Verwendung von Stereoisomerengemischen mit einem hohen 1,4-cis- bzw. einem hohen 1,3-trans-Isomerenanteil, auch andere Modifizierungsreaktionen wie z.B. die Trimerisierung unter Ausbildung von Isocyanurat- sowie von monocyclischen Iminooxadiazindionstrukturen (vgl. DE-A 19 611 849), die Carbodiimidisierung/Uretoniminbildung sowie die Dimerisierung unter Ausbildung von Uretdionstrukturen möglich sind, können die erfindungsgemäßen polycyclischen Iminooxadiazindione auch im Gemisch mit den genannten und weiteren Isocyanat-Folgeprodukten des Standes der Technik, wie z.B. Harnstoffe, Biurete, Urethane und/oder Allophanate, anfallen oder auch im nachhinein mit ihnen abgemischt werden. Weiterhin können die erfindungsgemäßen polycyclischen Iminooxadiazindione neben (Cyclo)polymerisaten ($\alpha$-Nylonverbindungen) anfallen, von denen sie gegebenenfalls, z.B. durch Extraktion oder Destillation, abgetrennt werden können. Die erfindungsgemäßen polycyclischen Iminooxadiazindione können auch in reiner Form erhalten werden, indem man von 1,4-Diisocyanaten und insbesondere im Fall der cycloaliphatischen Vertreter, von reinen trans-1,4-Diisocyanatocycloalkanen bzw. von reinen cis-1-Isocyanato-3-(1-isocyanatoalkyl)derivaten ausgeht und die entstehenden Produkte bzw. Produktgemische nach einem oder mehreren an sich bekannten Verfahren des Standes der Technik, wie z.B. Destillation, Dünnschichtdestillation, Molekulardestillation, Kristallisation oder Extraktion reinigt.

Für die Herstellung der erfindungsgemäßen polycyclischen Iminooxadiazindione A eignen sich viele der in der Literatur zur Herstellung von Oligoisocyanaten mit Isocyanurat-, monocyclischer Iminooxadiazindion- und/oder Uretdionstruktur zitierten Verbindungen. Bevorzugt werden, insbesondere wenn bei Temperaturen unterhalb ca. 120°C gearbeitet werden soll, weiche Lewis-Basen wie Alkylphosphine, z.B. Tributyl- oder Trioctylphosphin (jeweils gegebenenfalls als Isomerengemische) sowie Dialkylaminopyridine wie z.B. 4-(N,N-Dimethylamino)pyridin ('DMAP') in freier Form (Homogenkatalyse, vgl. H. J. Laas et al., J. Prakt. Chem. 336 (1994), 192 - 198) oder, gegebenenfalls chemisch modifiziert, auf Trägern fixiert eingesetzt (Heterogenkatalyse, vgl. EP 0 447 516 A1 oder WO 93/18014). Hierbei können auch weitere Zuschlagstoffe zum Einsatz kommen, die z.B. eine positive Auswirkung auf die Farbzahl der resultierenden Produkte haben (vergl. z.B. EP-A 735 027).

Die prinzipielle Eignung dieser Katalysatoren ganz allgemein zur Oligomerisierung von Isocyanaten unter Bildung bekannter Derivate wie Isocyanurate und/oder Uretdione ist seit langem bekannt (vgl. DE-A 1 670 720 sowie DE-A 3 739 549). Ihre Verwendung für die Herstellung der erfindungsgemäßen polycyclischen Iminooxadiazindione wurde jedoch nirgendwo erwähnt. Zwar wird in der DE-A 1 670 720 in einem Nebensatz (S. 5, Zeilen 1-5) die prinzipielle Möglichkeit einer Bildung von "Alkyliminodialkyloxadiazindionen" neben Carbodiimiden und Uretoniminen unter bestimmten Reaktionsbedingungen erwähnt (hohe Temperatur, niedrige Katalysatorkonzentration, diese Behauptungen treffen allerdings nur z.T. zu, vgl. die Argumentation in der DE-A 19 611 849 sowie die dort aufgeführten Vergleichsbeispiele), es findet sich in der Literatur jedoch nirgendwo ein Hinweis auf die erfindungsgemäßen bi- bzw. polycyclischen Iminooxadiazindione, noch auf die Sonderrolle, die 1,3- (vgl. DE-A 19 532 060) sowie 1,4-Diisocyanaten (vorliegende Erfindung) bei deren Bildung zukommt. In DE-A 3 739 549 wird beispielsweise der Einsatz (cyclo)aliphatischer Diisocyanate mit 6 bis 15 C-Atomen beansprucht, die durch Einsatz von 4-Dialkylaminopyridinen (z.B. DMAP) mit 99%-iger Selektivität in Polyisocyanate mit reiner Uretdionstruktur überführt werden können. Der einfachste Vertreter der in der vorliegenden Erfindung beschriebenen polycyclischen Iminooxadiazindione, das aus 2 Molekülen 1,4-Butandiisocyanat (BDI) aufgebaute 10-(4-Isocyanatobutyl)-8-oxa-1,6,10-triazabicyclo[$5.4.0^{1.7}$]undec-6-en-9,11-dion **D** der Formel (IV)

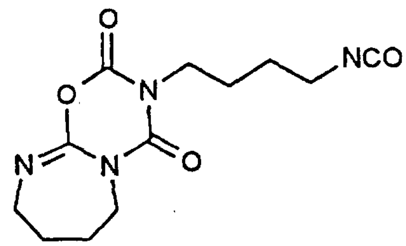

(IV)

D

fiele somit nicht einmal formal unter die Ansprüche der DE-A 3739549.

Der Fachmann konnte mithin aus den Literaturangaben keinerlei Hinweise auf eine mögliche Eignung der vorstehend genannten oder auch anderer Katalysatoren für die Bildung der erfindungsgemäßen polycyclischen Iminooxadiazindione, gegebenenfalls auch im Gemisch mit anderen Isocyanatfolgeprodukten, entnehmen.

Die Reaktion kann in Substanz, gegebenenfalls aber auch unter Verwendung eines gegenüber Isocyanatgruppen inerten Lösungsmittels und gegebenenfalls bei einem anderen als dem Normaldruck, im Temperaturbereich zwischen 0°C und der Zersetzungstemperatur des/der zugrundeliegenden (Di)isocyanat(gemisch)es durchgeführt werden. Bevorzugt wird die Herstellung der erfindungsgemäßen polycyclischen Iminooxadiazindione im Temperaturbereich von -30°C und 500°C durchgeführt.

Die Reaktion kann nach einem beliebig gewählten Umsetzungsgrad der Ausgangsverbindung(en) nach an sich bekannten Verfahren des Standes der Technik (z.B. J. Prakt. Chem. 336 (1994), 192 - 198) abgestoppt werden, beispielsweise durch Zugabe eines Katalysatorgiftes und/oder durch thermische Desaktivierung des Katalysators. Man kann die Reaktion aber auch bis zum im wesentlichen vollständigen Umsatz der monomeren 1,4-Diisocyanatoalkane durchführen und die so erhaltenen Produkte direkt in den weiter unten beschriebenen Anwendungen einsetzen. Anderenfalls kann anschließend von verbliebenen, nicht umgesetzten Monomeren nach einer Methode des Standes der Technik wie z.B. Destillation, Dünnschichtdestillation oder Extraktion abgetrennt werden.

Nach einer besonderen, gegebenenfalls kontinuierlich arbeitenden Ausführungsform des Verfahrens kann die Reaktion gegebenenfalls unter Zuhilfenahme eines bezüglich der erfindungsgemäßen Reaktion inerten Verdünnungsmittels, in einem Rohrreaktor vorgenommen werden. Hierbei sind Temperaturen des Reaktionsmediums von 50 - 550°C bevorzugt.

Weiterhin kann die Herstellung der erfindungsgemäßen polycyclischen Iminooxadiazindione auch in der Gasphase an einem geeigneten Kontakt erfolgen. Die für diese Ausführungsform des Verfahrens optimalen Reaktionstemperaturen richten sich nach dem (den) Siedepunkt(en) des Ausgangsisocyanat(gemisches) und liegen zwischen 100 und 500°C. Der hierbei angewendete Druck liegt im Bereich zwischen 1 mbar und 5 bar.

Die erfindungsgemäßen polycyclischen Iminooxadiazindione können, gegebenenfalls im Gemisch mit anderen bekannten Isocyanatfolgeprodukten, durch die üblichen Verfahren des Standes der Technik isoliert werden, wie z. B. Dünnschichtdestillation, Extraktion, Kristallisation oder Molekulardestillation und fallen dabei als farblose oder schwach gefärbte Flüssigkeiten oder Feststoffe an. Letztere weisen, abhängig von den eingesetzten Isocyanat(gemisch)en, einen Schmelzbereich von ca. 30-190 °C auf.

Die erfindungsgemäßen polycyclischen Iminooxadiazindione sind äußerst wertvolle Rohstoffe, die sowohl als Zwischenprodukt zur Herstellung bzw. Formulierung von Wirkstoffen als auch für die Verwendung im Kunststoff- und Lacksektor geeignet sind.

Bei den Vertretern, die aus, gegebenenfalls verzweigten, offenkettig aliphatischen 1,4-Diisocyanaten zugänglich sind (bicyclische Iminooxadiazindione, z.B. **D**, vgl. auch Beispiele 2-4) handelt es sich um niedrigviskose Flüssigkeiten, die sich, zusätzlich zu den weiter oben bereits angesprochenen Vorteilen, hervorragend als sogenannte Reaktivverdünner für den Einsatz in 'high solids' Polyurethanlacken eignen. Hier profitiert man ebenfalls von der Tatsache, daß ein Teil der Isocyanatgruppen in gewissermaßen 'selbstblockierter Form' im Iminooxadiazindionring vorliegt und der Reaktion mit dem zur Aushärtung des Kunststoffes bzw. der Beschichtung nötigen Reaktionspartner erst unter geigneten Reaktionsbedingungen, z.B. durch Erhöhung der Temperatur und/oder durch den Einsatz von Katalysatoren zugänglich gemacht wird (vgl. Beispiele 6-8).

Die erfindungsgemäßen polycyclischen Iminooxadiazindione können rein oder in Verbindung mit anderen Polyisocyanaten des Standes der Technik, z.B. mit Uretdion-, Biuret-, Allophanat-, Isocyanurat-, Urethan- sowie Carbodiimid-Strukturelementen, deren freie NCO-Gruppen gegebenenfalls mit typischen Blockierungsmitteln desaktiviert wurden, eingesetzt werden. Es ist dabei belanglos, ob die erfindungsgemäßen polycyclischen Iminooxadiazindione bei der Herstellung bereits in Abmischung mit den genannten Polyisocyanaten anderer Struktur entstehen, oder ob

sie im nachhinein erst mit letzteren vermengt werden.

Bevorzugt werden Gemische von polycyclischen Iminooxadiazindionen mit Polyisocyanaten vom Uretdion- ('Dimerisat-') und/oder Isocyanurat- ('Trimerisat-') und/oder monocyclischen Iminooxadiazindion- ('asymmetrisches Trimerisat'-) Strukturtyp eingesetzt, die gegebenenfalls freie NCO-Gruppen, letztere gegebenenfalls in blockierter Form enthalten können, wobei der Gehalt an polycyclischem Iminooxadiazindion 10 mol-% in der Mischung nicht unterschreitet (vgl. auch Beispiele 2-4).

Der besondere Vorteil der erfindungsgemäßen polycyclischen Iminooxadiazindione ist zum einen darin zu sehen, daß sie selbst bei längerer thermischer Belastung keine Rückspalttendenz in die zugrundeliegenden (monomeren) (Di)isocyanate aufweisen (vgl. auch Beispiel 5), jedoch andererseits eine ausreichend hohe, gegebenenfalls abgestufte Reaktivität gegenüber Verbindungen, die Zerevitinov-aktiven Wasserstoff enthalten, besitzen. Diese Beobachtungen sind insofern überraschend, als von den isomeren Isocyanuraten bekannt ist, daß dort das heterocyclische Ringsystem äußerst reaktionsträge ist. In der Literatur findet sich weiterhin nur ein Hinweis darauf, daß der Iminooxadiazindionring einer Solvolysereaktion unterworfen werden kann (Chem. Ber., 60 (1927), 295).

Befinden sich andere reaktive Zentren, beispielsweise Isocyanatgruppen, unter bzw. in den unter Anspruch 1 genannten Substituenten $R^1$ - $R^9$, bieten sich die erfindungsgemäßen polycyclischen Iminooxadiazindione für Anwendungen mit dualem Vernetzungsmechanismus an. Hierfür setzt man beispielsweise die freie(n) reaktiven Gruppen, z.B. Isocyanatgruppen, in einem ersten Reaktionsschritt mit einer Komponente $Z(OH)_n$ bzw. $Z[N(R')H]_n$ um (vgl. Schema 1) und führt in einem unabhängigen, gegebenenfalls katalysierten, zweiten Schritt die Vernetzung unter Abbau der Iminooxadiazindionstruktur durch. Hierbei zeigt sich, daß bis zu zwei Reaktionspartner, die Zerevitinov-aktiven Wasserstoff enthalten, je Equivalent Iminooxadiazindioneinheit gebunden werden können (Schema 1).

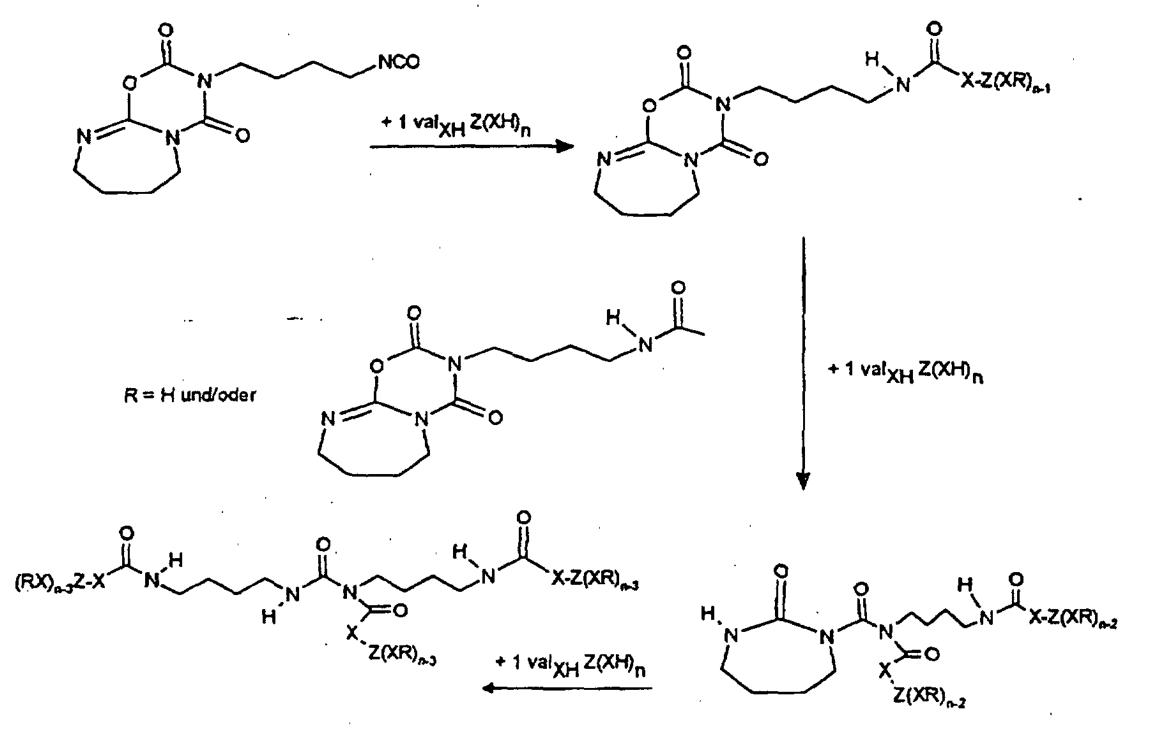


Z = n-wertige(r) Alkyl-, Cycloalkyl-, Aralkyl- und/oder Arylrest(e), wie sie z.B. aus, gegebenenfalls aminofunktionellen Polyacrylat(en), Polyester(n), Polycarbonat(en), Polyether(n) und/oder Mono- sowie Polyolen durch Entfernung der OH- und/oder N(R')H-Gruppen erhalten werden und beliebige Gemische derselben zur Einstellung einer mittleren Funktionalität (Vernetzungsdichte) aber auch H ($Z(XH)_n = H_2O$, $NH_3$, $N_2H_4$)

X = O,NR'

R' = H, Alkyl, Cycloalkyl, Aralkyl, Aryl sowie die unter Z aufgeführten Gruppen

n = 1-6

Schema 1: Kettenverlängerungs- und Vernetzungsreaktionen mit den erfindungsgemäßen polycyclischen Iminooxadiazindionen hier beispielhaft an der Umsetzung des einfachsten Vertreters D dargestellt.

Durch die simultane Anwesenheit anderer reaktiver Zentren im Molekül der erfindungsgemäßen polycyclischen Iminooxadiazindione oder durch eine entsprechende Modifizierung im o.g. "Vorverlängerungsschritt" bei Gegenwart einer mehr als monofunktionellen Komponente $Z(XH)_n$ ($n > 1$), läßt sich deshalb prinzipiell jede gewünschte Funktionalität einstellen. Die im ersten Reaktionsschritt erhaltenen Produkte können auch zur einkomponentigen Weiterverarbeitung verwendet werden, wobei man die Vernetzung (Aushärtung) zum gebrauchsfähigen Endprodukt durch thermisch und/oder katalytisch induzierten Abbau der Iminooxadiazindionstruktur herbeiführt. Derartige Produkte lassen sich beispielsweise mit gutem Erfolg als Einkomponenten-Polyurethan-Pulverlacksysteme einsetzen.

Selbstverständlich kann die Aushärtung zur gebrauchsfähigen Beschichtung bzw. zum Kunststoff auch in einem Schritt geschehen (2 K-Technologie).

Die resultierenden Produkte, unabhängig davon, ob sie nach einem ein- oder zweikomponentigen Aufbauweg hergestellt wurden, weisen das für Polyurethansysteme typische, hohe Eigenschaftsprofil auf.

Neben den erfindungsgemäßen Verfahrensprodukten und den gegebenenfalls mitverwendeten weiteren Bindemittelkomponenten und Lacklösungsmitteln oder Lacklösungsmittelgemischen wie beispielsweise Toluol, Xylol, Cyclohexan, Chlorbenzol, Butylacetat, Ethylacetat, Ethylglykolacetat, Methoxypropylacetat, Aceton, Testbenzin, höher substituierte Aromaten (Solventnaphtha$^R$, Solvesso$^R$, Shellsol$^R$, Isopar$^R$, Nappa$^R$, Diasol$^R$) können in den Beschichtungen auch weitere Hilfs- und Zusatzmittel verwendet werden, wie z.B. Benetzungsmittel, Verlaufsmittel, Hautverhinderungsmittel, Antischaummittel, Mattierungsmittel, viskositätsregulierende Stoffe, Katalysatoren für die NCO-OH/NH-Reaktion und/oder die Öffnung des Iminooxadiazindionringes, Pigmente, Farbstoffe, UV-Absorber und Stabilisatoren gegen thermische und oxidative Einflüsse.

Die Beschichtungsmittel auf Basis der erfindungsgemäßen polycyclischen Iminooxadiazindione können zur Beschichtung einer Vielzahl von Materialien dienen, wie z.B. Holz, Kunststoff, Leder, Metall, Papier, Beton , Mauerwerk, Keramik und Textil.

Alle Prozentangaben sind, soweit nicht anders vermerkt, als Masseprozent zu verstehen.

## Beispiele

### Beispiel 1

(Vergleichsbeispiel, s. auch EP-A 571 038, Beispiele 1-3, sowie EP-A 57 653)

Jeweils 200 g (1.43 mol) 1,4-Diisocyanatobutan (im weiteren: BDI) werden bei 60°C (a-c) bzw. 120°C (d) in einem 250 ml Vierhalskolben, versehen mit mechanischem Rührer, Innenthermometer, Rückflußkühler und Dosiereinrichtung für den Katalysator, unter Rühren vorgelegt und anschließend portionsweise über einen Zeitraum von 2-3 Stunden unter externer Heizung bzw. Kühlung, um die Reaktionstemperatur im Bereich zwischen 60 und 70°C (a-c), bzw. 120°C (d) zu halten mit jeweils insgesamt:

a) 25 mg Triton® B (Benzyltrimethylammoniumhydroxid, Aldrich), gelöst in 2-Ethyl-1,3-hexandiol,

b) 55 mg Polycat® 41, (N, N', N"-Tris-(dimethylaminopropyl)hexahydrotriazin, Air Products), gelöst in n-Hexan,

c) 8 g DABCO® (1.4-Diaza-[2.2.2]bicyclooctan, Air Products), gelöst in Butanol, bzw.

d) 3 g HMDS (Bis(trimethylsilyl)amin, Aldrich), unverdünnt,

versetzt. Nach Erreichen eines titrimetrisch (DIN 53 185) ermittelten NCO-Gehaltes der Reaktionslösung von ca. 53 % wird durch Zugabe der zur eingesetzten Katalysatormenge jeweils stöchiometrischen Menge Di-n-butylphosphat (bei a)-c)) bzw. $H_2O$ (bei d)) die weitere Reaktion unterbunden ('abgestoppt'). Anschließend werden die Reaktionsmischungen mittels Gelpermeationschromatographie, im weiteren GPC, analysiert, vgl. Tab. 1:

Tabelle 1

| Versuch | Polymeranteil (> 2500 g/mol)[1) ] [Fl-%][2)] | oligomere Isocyanurat-Polyisocyanate[1)] [Fl-%][2)] | Andere[1)] [Fl-%][2)] | **D** [Fl-%][2)] |
|---|---|---|---|---|
| 1a) | ca. 60 | ca. 18 | ca. 22 | n.n. |
| 1b) | ca. 13 | ca. 82 | ca. 5 | n.n. |
| 1c) | ca. 29 | ca. 55 | ca. 15 | 0.8 |
| 1d) | n.n. | ca. 90 | ca. 9 | 0.6 |

1) die Molgewichtsverteilung der polymeren Anteile variiert bei den Versuchen etwas, das Mittel liegt meißt oberhalb 10 000 g/mol, zur Struktur dieser Polymeren sind keine Untersuchungen angestellt worden (vgl. auch Y. Iwakura, K. Uno and K. Ichikawa, Journal of Polymer Science: Part A, 2 (1964) 3387-3404), neben Isocyanurat-Polyisocyanaten sind vor allem Uretdiongruppen enthaltende Spezies sowie Folgeprodukte der Katalysatoren bzw. der Katalysatorlösungsmittel (zusammengefaßt unter Andere) vorhanden,

2) Fl.-% = Flächenprozent lt. GPC ohne Monomer (nicht normiert), da bis auf **D** alle Spezies in Form mehr oder weniger ausgeprägter, sich teilweise gegenseitig überlagernder Oligomerenreihen anfallen, ist eine genauere Angabe nicht möglich, n.n. = nicht nachweisbar

Da eine dünnschichtdestillative Abtrennung des überschüssigen Monomers nur bei d) möglich ist (hohe Polymeranteile bei a) - c), vgl. Tab. 1) wurden die nahezu polymerfreien Harze bei den drei erstgenannten Versuchen, wie in der EP-A 0 571 038 beschrieben, durch Extraktion gewonnen und nach dünnschichtdestillativer Abtrennung von Extraktionsmittel und mitextrahiertem Monomer erneut analysiert. Die NCO-Gehalte lagen zwischen 27.3 und 29.2 % bei dynamischen Viskositäten der Harze, gemessen bei 23°C, um 5 000 mPa·s bei a) - c), bzw. um 15 000 mPa·s im Fall von d). IR- und NMR-spektroskopisch konnte die Dominanz der Isocyanuratstruktur eindeutig belegt werden. In keinem der Fälle wurde ein molarer Anteil an **D** über 5 mol-% gefunden.

**Beispiel 2 (erfindungsgemäß)**

Es wird wie in Beispiel 1 verfahren, mit der Ausnahme, daß 500 g BDI unter portionsweiser Zugabe von 1.0 - 1.2 g Tributylphosphin (Hoechst) bei:

a) 90°C
b) 60°C
c) 60°C
d) 30°C

oligomerisiert werden und die Reaktion bei einem titrimetrisch bestimmten NCO-Gehalt der Mischung von:

a) 47.9 %,
b) 47.9 %,
c) 21.0 % bzw.
d) 43.5 %

durch Zugabe von:

a) 0.75 g p-Toluolsulfonsäuremethylester (TSE) und anschließendem einstündigen Erhitzen auf 80°C,
b) - d) jeweils 0.06 g elementarem Schwefel

abgestoppt werden. Nach dünnschichtdestillativer Aufarbeitung der beiden ersten Ansätze bei 100°C/0.2 mbar erhält man jeweils 173 g an nahezu farblosen Harzen mit einem NCO-Gehalt von a): 21.6 bzw. b): 18.9 % und einer Viskosität von a): 600 bzw. b): 1960 mPa·s (23°C). Der Gehalt an monomerem BDI beträgt bei a) und b) 0.2 % (GC-Analyse), bei c) ca. 10 % (GPC-Analyse) und bei d) ca. 42 % (GPC-Analyse).

Der durch GPC bestimmte Gehalt an bicyclischem Iminooxadiazindion **D** in den oligomeren Produkten (d.h.: ohne Berücksichtigung des Monomeranteils bei c) und d)) beläuft sich auf :

a) ca. 36 Fl.-%,
b) ca. 49 Fl.-%,

c) ca. 39 Fl.-% bzw.
d) ca. 48 Fl.-%.

Die Identität von **D** folgt zweifelsfrei aus den Daten der Elementaranalyse (zur Charakterisierung der Substanz vgl. auch Beispiel 4), der GPC, GC-MS sowie der $^1$H-, $^{13}$C-NMR- und der IR-Spektroskopie. Der NMR-spektroskopisch ermittelte molare Anteil der Iminooxadiazindiongruppen an den NCO-Folgeprodukten der Harze beläuft sich auf:

a) ca. 43 mol-%,
b) ca. 59 mol-%,
c) ca. 69 mol-% bzw.
d) ca. 75 mol-%.

**Beispiel 3 (erfindungsgemäß)**

173 g des Harzes aus Beispiel 2a) werden bei einer Temperatur des Heizmediums von 180°C und einem Druck von 0.2 mbar der Dünnschichtdestillation unterworfen. Hierbei destillieren 134 g einer farblosen Flüssigkeit ab, die einer erneuten Dünnschichtdestillation zur Abtrennung des infolge (Uretdion)spaltung bei der hohen Destillationstemperatur gebildeten monomeren BDI bei 120°C/0.2 mbar unterworfen werden. Es resultieren 69 g monomerenfreies Produkt mit einem NCO-Gehalt von 22.3 % und einer dynamischen Viskosität von 640 mPa·s (23°C), das neben ca. 60 % Uretdion **E** der Formel (V) im wesentlichen reines **D** der Formel (IV) enthält.

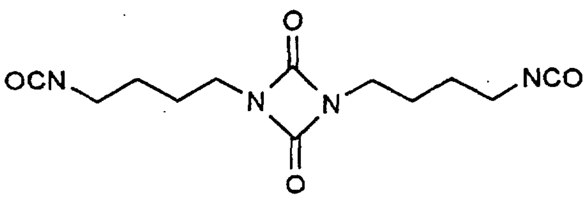

(V)

E

**Beispiel 4 (erfindungsgemäß)**

Es wird wie in Beispiel 1 verfahren mit der Ausnahme, daß 100 g BDI, gelöst in 900 g Toluol, unter Zugabe. von 6 g Tributylphosphin (Hoechst) bei 60°C bis zu einem titrierten NCO-Gehalt der Mischung von 4.21 % umgesetzt werden, durch Zugabe von 1 g elementarem Schwefel abgestoppt wird und anschließend am Rotationsverdampfer bei 50°C/5 mbar der größte Teil Toluol abgezogen wird. Man erhält 114.8 g einer Flüssigkeit mit einem NCO-Gehalt von 33.8 %. Die NMR-spektroskopische Analyse letzterer belegt, daß ca. 85 mol-% der NCO-Folgeprodukte die erfindungsgemäße bicyclische Iminooxadiazindionstruktur aufweisen. Nach destillativer Abtrennung des nicht umgesetzten Monomeren wird die zur titrierten NCO-Äquivalentmenge stöchiometrische Menge einer Methylaminlösung in Toluol bei Zimmertemperatur zugetropft und nach vollständiger Reaktion der NCO-Gruppen (IR) langsam eingeengt. Bei beginnender Kristallisation wird das Einengen unterbrochen, über Nacht im Kühlschrank gelagert und anschließend der ausgefallene Niederschlag abgesaugt, mehrmals mit wenig kaltem Ether gewaschen und getrocknet. Die Elementaranalyse des Monomethylharnstoffes von **D** lieferte folgendes Ergebnis: gef. (ber. für $C_{13}H_{21}N_5O_4$): C: 50.21 % (50.15 %), H: 6.92 % (6.80 %), N: 22.48 % (22.49 %).

**Beispiel 5**

(erfindungsgemäß, Anwendungsbeispiel zur Thermostabilität der polycyclischen Iminooxadiazindione)

10 g eines Polyisocyanatgemisches, erhalten nach der in Beispiel 3 beschriebenen Arbeitsweise (molares Verhältnis von Iminooxadiazindion zu Uretdion = 0.7), werden im Verlauf von 50 min in einer Standard-Destillationsapparatur unter Durchleiten eines feinperligen Stickstoffstromes mittels einer nicht zu feinen Siedekapillare von 150°C auf 180 °C bei langsam von 2 auf 0.1 mbar verringertem Druck erhitzt. Das übergegangene Destillat (5.0 g) besteht lt. gaschromatographischer Analyse zu über 95 % aus BDI ($n_D^{20}$ = 1.4518). Der Destillationsrückstand wird anschließend bei 200 - 250°C/0.5 mbar einer Kugelrohrdestillation unterworfen. Man erhält 3.33 g einer farblosen Flüssigkeit. Die NMR-spektroskopische Analyse zeigt, daß das molare Verhältnis von Iminooxadiazindion zu Uretdion auf 2.6 angestiegen ist.

Dieser Versuch belegt, daß das BDI-Dimer **D** mit bicyclischer Iminooxadiazindionstruktur im Gegensatz zum isomeren Uretdion-Dimer **E** keine nachweisbare Tendenz zur thermisch induzierten Rückspaltung in das zugrundeliegende Ausgangsdiisocyanat (BDI) aufweist. Mithin sind die erfindungsgemäßen Iminooxadiazindione thermisch äußerst rückspaltstabil.

**Beispiel 6**

(erfindungsgemäß, Anwendungsbeispiel: Modellreaktionen zur Vernetzung nach Schema 1)

Jeweils 15 g eines Polyisocyanatgemisches aus Bsp. 2b) werden unter Rühren mit:

a) 5 g n-Butanol,
b) 5 g n-Butanol und 50 mg Dibutylzinndilaurat,
c) 10 g n-Butanol bzw.
d) 10 g n-Butanol und 50 mg Dibutylzinndilaurat

versetzt und nach Abklingen der Exothermie zwei Stunden bei 50°C, a) und b), bzw. am schwachen Rückfluß (Badtemperatur 130°C), c) und d), gehalten. Anschließend wird analysiert. Bei a) ist sowohl nicht umgesetztes NCO als auch freies Butanol nachweisbar, im IR-Spektrum von b) fehlt die NCO-Bande gänzlich, der eingesetzte Alkohol liegt vollständig als Urethan gebunden vor. Das molare Verhältnis von Iminooxadiazindion- zu Uretdiongruppen ist in beiden Fällen gegenüber dem Ausgangswert nahezu unverändert.

Bei c) und d) treten keine NCO-Banden im IR-Spektrum auf. Der Iminooxadiazindionring läßt sich nur im Fall von c) gerade noch NMR-spektroskopisch nachweisen wohingegen in beiden Fällen, c) und d), noch deutliche Uretdion-Signale sichtbar sind. Weiterhin werden die aus der Ringöffnungsreaktion gemäß Schema 1 entstandenen Spezies detektiert.

**Beispiel 7**

(erfindungsgemäß, Anwendungsbeispiel zur zweikomponentigen Verarbeitungsweise)

10 g eines Polyisocyanatgemisches, erhalten nach der in Beispiel 2a) beschriebenen Arbeitsweise, werden mit 11.1 g eines OH-funktionellen Polyesters auf Basis Phthalsäureanhydrid/Trimethylolpropan der OH-Zahl 260 (Equivalentgewicht: 215 g/$val_{OH}$) in 15 g n-Butylacetat vermischt (NCO : OH-Verhältnis = 1:1), in einer 180 µm dicken Schicht auf eine Glasplatte aufgetragen und einer forcierten Trocknung für 30 Minuten bei 80 °C unterworfen. Man erhält einen Lackfilm, der nach 100 Doppelhüben mit MEK deutlich angelöst wird.

Verwendet man einen 50%igen Überschuß an OH-funktioneller Polyesterkomponente, was bei der Verwendung klassischer Isocyanathärter in einer deutlichen Verschlechterung der Lackeigenschaften der erhaltenen Beschichtung resultieren würde, und führt die Vernetzung für 60 Minuten bei 130°C durch, erhält man einen klaren Lackfilm, der auch nach 150 Doppelhüben mit MEK keine Anzeichen einer Anlösung zeigt. Dieser Umstand ist der besseren Vernetzung zuzuschreiben, die infolge Öffnung der Iminooxadiazindionringe bei höherer Temperatur einsetzt (vgl. Schema 1).

## Patentansprüche

**1.** Polycyclische Iminooxadiazindione der allgemeinen Formel (I) A

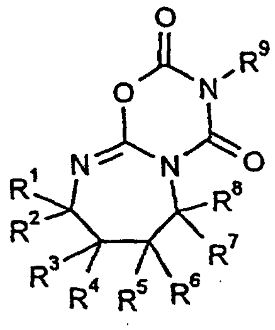

(I),

A

in denen $R^1$-$R^8$ für gleiche oder verschiedene Reste wie: H, Alkyl, Cycloalkyl, Aralkyl und Aryl, die ihrerseits funktionelle Substituenten tragen können, die nicht mit Isocyanatgruppen reagieren, wie z.B. Isocyanato(cyclo)alkyl, Halogen, Oxo (-C(=O)-), Alk- oder Aryl-oxi (R-O-), Alkoxycarbonyl (ROC(=O)-), Aroyl (RC(=O)-) usw., wobei auch $R^1$ und/oder $R^2$ untereinander sowie mit einem oder mehreren Substituenten aus der Reihe $R^3$ - $R^8$ sowie den entsprechenden Atomen des Siebenringsegmentes in A einen oder mehrere, gegebenenfalls partiell ungesättigte, Ringe bilden können und $R^3$ oder $R^4$ mit wahlweise $R^5$ oder $R^6$ sowie den beiden nicht stickstoffgebundenen Kohlenstoffatomen des Siebenringsegmentes in A einen aromatischen Ring bilden können und schließlich $R^9$ beschrieben wird durch: H, Alkyl, Cycloalkyl, Aralkyl, Aryl, funktionell substituierte (cyclo)aliphatische, heterocyclische und/oder aromatische Reste die z.B. eine oder mehrere Isocyanatgruppen und/oder andere funktionelle Substituenten, die nicht mit Isocyanaten reagieren, wie z.B. Halogen, Oxo (-C(=O)-), Alk- oder Aryl-oxi (R-O-), Alkoxycarbonyl (ROC(=O)-), Aroyl (RC(=O)-) usw. enthalten können.

**2.** Polycyclische Iminooxadiazindione gemäß Anspruch 1, in denen $R^1$-$R^8$ die in Anspruch 1 beschriebene Bedeutung haben und $R^9$ für einen, gegebenenfalls substituierten, gegebenenfalls Heteroatome enthaltenden Alkyl-, Cycloalkyl-, Aralkyl- oder Arylsubstituenten beispielsweise B, steht.

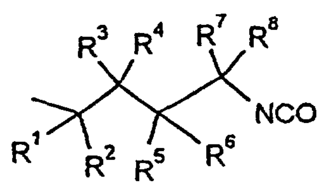

B

**3.** Bicyclische Iminooxadiazindione gemäß Anspruch 1, **dadurch gekennzeichnet, daß** sie durch Dimerisierung zweier, gegebenenfalls verzweigter, offenkettiger aliphatischer Diisocyanate in denen die beiden Isocyanatgruppen in 1,4-Stellung zueinander angeordnet sind, hergestellt wurden.

**4.** 10-(4-Isocyanatobutyl)-8-oxa-1.6.10-triazabicyclo[5.4.0$^{1.7}$]undec-6-en-9.11-dion **D**

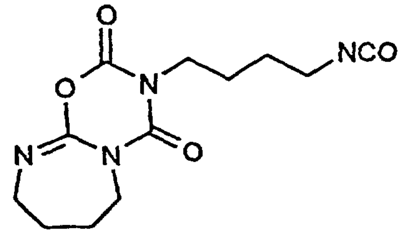

D

5. Gemische von Verbindungen gemäß Anspruch 1-4 mit anderen Isocyanat-Folgeprodukten wie z.B. (Poly)isocyanat-Isocyanuraten, -Uretdionen, -Biureten, -Allophanaten, -Carbodiimiden sowie -Urethan-Prepolymeren, die gegebenenfalls NCO-Gruppen, letztere gegebenenfalls in blockierter Form enthalten, **dadurch gekennzeichnet, daß** mindestens 10 mol-% der Komponenten der Mischung Verbindungen des Strukturtyps gemäß Anspruch 1 - 4 darstellen.

6. Verfahren zur Herstellung der Polycyclischen Iminooxadiazindione der Formel **A**, **dadurch gekennzeichnet, daß** Di- oder Oligoisocyanate der Formel **C** in welcher $R^1$ - $R^8$ die bei Formel **A** angegebene Bedeutung haben, gegebenenfalls im Gemisch mit anderen $C_1$ - $C_{30}$ Mono- und/oder Polyisocyanaten, gegebenenfalls in einem Lösungsmittel oder in der Gasphase, in Anwesenheit eines Katalysators zu den erfindungsgemäßen polycyclischen Iminooxadiazindionen der Formel **A** umgesetzt werden.

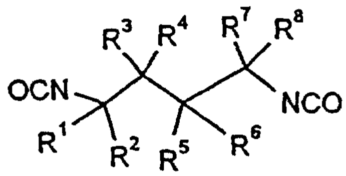

**C**

7. Die Herstellung der Verbindungen gemäß Anspruch 1 nach einem Verfahren gemäß Anspruch 6, **dadurch gekennzeichnet, daß** die katalytisch induzierte Reaktion, gegebenenfalls in Gegenwart eines Lösungsmittels in kondensierter Phase oder ingegenwart eines inerten Verdünnungsmittels in der Gasphase bei Temperaturen zwischen 0 und +500°C durchgeführt wird.

8. Die Verwendung der gemäß Anspruch 1-5 definierten Verbindungen als Komponente oder Zwischenprodukt zur Herstellung von, gegebenenfalls geschäumten, Polyurethankunststoffen, Lacken und Beschichtungsmitteln.

9. Die Verwendung der gemäß Anspruch 1-4 definierten Verbindungen als Zwischenprodukt zur Herstellung oder Formulierung von Wirkstoffen, pharmazeutischen Erzeugnissen.

## Claims

1. Polycyclic iminooxadiazinediones corresponding to the general formula (I) **A**

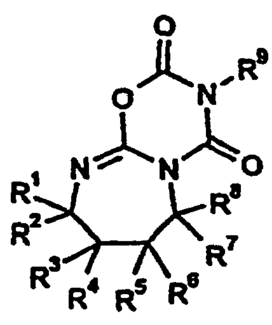

(I),

**A**

wherein $R^1$ to $R^8$ are identical or different groups such as: H, alkyl, cycloalkyl, aralkyl and aryl, which for their part may carry functional substituents which do not react with isocyanate groups such as, for example, isocyanato (cyclo)alkyl, halogen, oxo (-C(=O)-), alkoxy or aryloxy (R-O-), alkoxycarbonyl (ROC(=O)-), aroyl (RC(=O)-) et cetera, wherein also $R^1$ and/or $R^2$ can form one or more, optionally partially unsaturated, rings with one another as well as with one or more substituents from among $R^3$ to $R^8$ as well as with the corresponding atoms of the seven-membered ring segment in **A**, and $R^3$ or $R^4$ can form an aromatic ring with alternatively $R^5$ or $R^6$ as well as with

the two carbon atoms of the seven-membered ring segment in **A** which are not bonded to nitrogen, and finally $R^9$ denotes: H, alkyl, cycloalkyl, aralkyl, aryl, functionally substituted (cyclo)aliphatic, heterocyclic and/or aromatic groups which may contain, for example, one or more isocyanate groups and/or other functional substituents which do not react with isocyanates such as, for example, halogen, oxo (-C(=O)-), alkoxy or aryloxy (R-O-), alkoxycarbonyl (ROC(=O)-), aroyl (RC(=O)-) et cetera.

**2.** Polycyclic iminooxadiazinediones according to claim 1, wherein $R^1$ to $R^8$ have the meaning given in claim 1 and $R^9$ represents an alkyl, cycloalkyl, aralkyl or aryl substituent, for example **B**, optionally substituted and optionally containing hetero atoms.

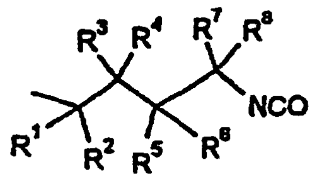

**B**

**3.** Bicyclic iminooxadiazinediones according to claim 1, **characterised in that** they have been prepared by dimerisation of two, optionally branched, open-chain aliphatic diisocyanates wherein the two isocyanate groups are arranged in the 1,4 positions to one another.

**4.** 10-(4-Isocyanatobutyl)-8-oxa-1,6,10-triazabicyclo[5.4.$0^{1.7}$]undec-6-en-9,11-dione **D**

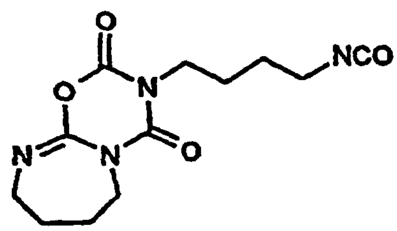

**D**

**5.** Mixtures of compounds according to claims 1 to 4 with other isocyanate derivatives such as, for example, (poly) isocyanate-isocyanurates, -uretdiones, -biurets, -allophanates, -carbodiimides and -urethane prepolymers, which optionally contain NCO groups, the latter optionally in blocked form, **characterised in that** at least 10 mol-% of the components of the mixture are compounds of the structural type according to claims 1 to 4.

**6.** A process for the preparation of polycyclic iminooxadiazinediones corresponding to formula **A**, **characterised in that** diisocyanates or oligoisocyanates corresponding to formula **C** wherein $R^1$ to $R^8$ have the meaning given for formula **A**, optionally in a mixture with other $C_1$-$C_{30}$ monoisocyanates and/or polyisocyanates, optionally in a solvent or in the gas phase, are reacted in the presence of a catalyst to form the polycyclic iminooxadiazinediones according to the invention corresponding to formula **A**.

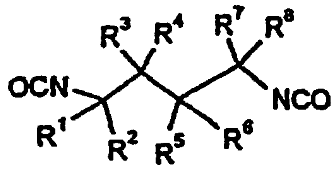

C

7. The preparation of the compounds according to claim 1 by a process according to claim 6, **characterised in that** the catalytically induced reaction, optionally in the presence of a solvent in condensed phase or in the presence of an inert diluent in the gas phase, is carried out at temperatures of between 0°C and +500°C.

8. The use of the compounds defined according to claims 1 to 5 as component or intermediate product for the production of optionally foamed, polyurethane plastics, paints and coatings.

9. The use of the compounds defined according to claims 1 to 4 as intermediate product for the production or formulation of active substances and pharmaceutical products.

**Revendications**

1. Iminooxadiazinediones polycycliques de formule générale (I) A

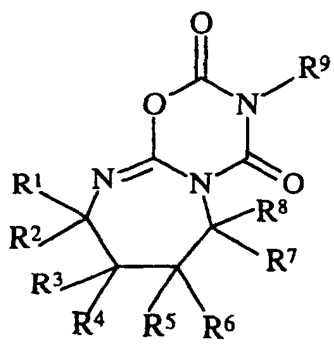

(I),

A

dans laquelle $R^1$ à $R^8$, ayant des significations identiques ou différentes, représentent : H, des groupes alkyle, cycloalkyle, aralkyle et aryle qui peuvent eux-mêmes porter des substituants fonctionnels ne réagissant pas avec les groupes isocyanate, par exemple des groupes isocyanato(cyclo)alkyle, des halogènes, des groupes oxo (-C(=O)-), alcoxy ou aryloxy (R-O-), alcoxycarbonyle (ROC(=O)-), aroyle (RC(=O)-) etc., $R^1$ et/ou $R^2$ pouvant également former entre eux ou avec un ou plusieurs substituants parmi $R^3$ à $R^8$ et les atomes correspondants du segment cyclique à sept chaînons de A un ou plusieurs cycles éventuellement saturés en partie, et $R^3$ ou $R^4$ peut former, avec $R^5$ ou $R^6$ à volonté et avec les deux atomes de carbone du segment cyclique à sept chaînons de A non fixés à l'azote, un cycle aromatique, et finalement $R^9$ représente H, un groupe alkyle, cycloalkyle, aralkyle, aryle, un radical (cyclo)aliphatique, hétérocyclique et/ou aromatique portant des substituants fonctionnels, qui peuvent contenir par exemple un ou plusieurs groupes isocyanate et/ou d'autres substituants fonctionnels ne réagissant pas avec les isocyanates, par exemple des halogènes, des groupes oxo (-C(=O)-), alcoxy ou aryloxy (R-O-), alcoxycarbonyle (ROC(=O)-), aroyle (RC(=O)-) etc.

2. Iminooxadiazinediones polycycliques selon la revendication 1, pour lesquelles $R^1$ à $R^8$ ont les significations indiquées dans la revendication 1 et $R^9$ représente un substituant alkyle, cycloalkyle, aralkyle ou aryle éventuellement substitué et contenant éventuellement des hétéroatomes, par exemple B

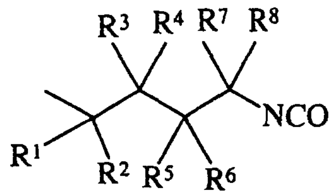

B

3. Iminooxadiazinediones bicycles selon la revendication 1, **caractérisées en ce qu'**elles ont été préparées par dimérisation de deux diisocyanates aliphatiques acycliques éventuellement ramifiés dans lesquels les deux groupes isocyanate sont en position mutuelle 1,4.

4. La 10-(4-isocyanatobutyl)-8-oxa-1,6,10-triazabicyclo[$5.4.0^{1,7}$]-undéc-6-ène-9,11-dione D

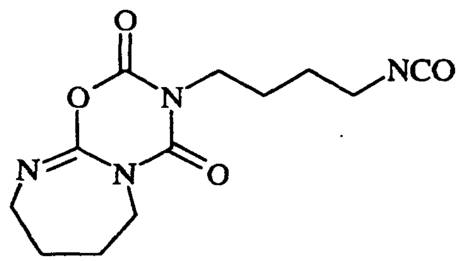

D

5. Mélanges de composés selon les revendications 1 à 4 et d'autres produits de conversion d'isocyanates, par exemple des (poly)isocyanate-isocyanurates, -uretdiones, -biurets, -allophanates, -carbodiimides et prépolymères de (poly)isocyanate-uréthannes contenant le cas échéant des groupes NCO, ces derniers éventuellement bloqués, **caractérisés en ce que** 10 mol % au moins des composants du mélange consistent en composés du type de structure selon les revendications 1 à 4.

6. Procédé de préparation des iminooxadiazinediones polycycliques de formule A, **caractérisé en ce que** l'on convertit des di- ou oligo-isocyanates de formule C dans laquelle $R^1$ à $R^8$ ont les significations indiquées en référence à la formule A, éventuellement en mélange avec d'autres mono- et/ou polyisocyanates en $C_1$-$C_{30}$, le cas échéant dans un solvant ou en phase gazeuse, en présence d'un catalyseur, en les iminooxadiazinediones polycycliques selon l'invention de formule A

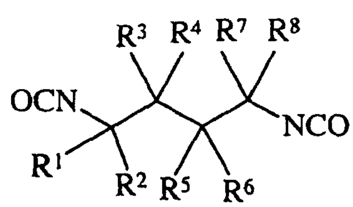

C

7. La préparation des composés selon la revendication 1 par un procédé selon la revendication 6, **caractérisée en ce que** la réaction, induite par un catalyseur, est réalisée éventuellement en présence d'un solvant en phase condensée ou en présence d'un diluant inerte en phase gazeuse à des températures de 0 à +500°C.

8. L'utilisation des composés définis dans les revendications 1 à 5 en tant que composants ou produits intermédiaires

pour la préparation de résines synthétiques de polyuréthanne éventuellement gonflées en mousse, de vernis et produits de revêtement.

9. L'utilisation des composés définis dans les revendications 1 à 4 en tant que produits intermédiaires de la préparation ou de la formulation de substances actives, produits pharmaceutiques.